# EUROPÄISCHE PATENTSCHRIFT

(11) **EP 3 030 188 B1**
(45) Veröffentlichungstag und Bekanntmachung des Hinweises auf die Patenterteilung: **16.05.2018**
(21) Anmeldenummer: 14747054.6
(22) Anmeldetag: 04.08.2014
(51) Int. Cl.: A61B 34/20

(54) **SYSTEM ZUR REKONSTRUKTION SYMMETRISCHER KÖRPERTEILE**
SYSTEM FOR RECONSTRUCTION OF SYMMETRICAL BODY PARTS
SYSTÈME POUR LA RECONSTRUCTION DE PARTIES DU CORPS SYMÉTRIQUES

(30) Priorität: 05.08.2013 DE 102013215395
(43) Veröffentlichungstag der Anmeldung: 15.06.2016
(73) Patentinhaber: Fiagon GmbH, 16761 Hennigsdorf (DE)
(72) Erfinder: KRÜGER, Timo, 13465 Berlin (DE)
(74) Vertreter: Eisenführ Speiser
(86) Internationale Anmeldenummer: PCT/EP2014/066754
(87) Internationale Veröffentlichungsnummer: WO 2015/018804

(56) Entgegenhaltungen:
- WO-A1-01/35842
- WO-A2-2004/110309
- US-A1- 2006 241 388
- US-A1- 2008 319 448
- US-A1- 2012 016 269

## Beschreibung

Die Erfindung betrifft ein System zur Unterstützung der Planung und/oder Durchführung der Rekonstruktion üblicherweise symmetrischer Körperteile eines Patienten.

Bei invasiven chirurgischen Eingriffen werden chirurgische Instrumente während einer Operation von einem Operateur in einem Operationsgebiet im Körper eines Patienten bewegt. Als Operationsgebiet wird der Raum im Inneren des Patienten bezeichnet, der während der Operation potenziell oder effektiv von den eingesetzten chirurgischen Instrumenten beeinträchtigt wird. Zur Operation zählen das Ein- und Ausführen von chirurgischen Instrumenten in den Körper des Patienten hinein bzw. aus dem Körper des Patienten heraus sowie das Bewegen der chirurgischen Instrumente im Körper des Patienten und der Einsatz der chirurgischen Instrumente in einem im Operationsgebiet angeordneten und genau vorbestimmten Eingriffsgebiet. Als Eingriffsgebiet wird der Teil im Operationsgebiet bezeichnet, der vom Operateur zu bearbeiten ist. Hierbei kann es sich z.B. um zu entfernendes Gewebe oder zu verschließende Gefäße handeln. Demnach kann ein Operationsgebiet eine Vielzahl von Eingriffsgebieten umfassen. Neben den Eingriffsgebieten liegt im Operationsgebiet eine Vielzahl von sensiblen Strukturen, die vor Beschädigungen durch die chirurgischen Instrumente zu bewahren sind. Zu den sensiblen Strukturen zählen z.B. Gefäße, Organe, Nerven, Muskeln, Bänder, Sehnen und sonstiges, generell intaktes Gewebe, das erhalten bleiben soll, um die Auswirkungen der Operation auf ein notwendiges Minimum zu beschränken, da jede weitere Beeinträchtigung des Körpers des Patienten während der Operation das Gesundheitsrisiko des Patienten erhöhen und das Operationsergebnis negativ beeinflussen kann.

Tumorerkrankungen, Unfälle oder angeborene Fehlbildungen erfordern oft eine Rekonstruktion des Gewebes des Patienten. Insbesondere im Gesichtsbereich sind dabei funktionale und ästhetische Aspekte bei der Herrichtung der Knochen- und Weichteilstruktur des Patienten zu berücksichtigen. Bei derartigen Eingriffen im Gesicht des Patienten ist es besonders wichtig, dass das Gesicht nach der Operation eine möglichst exakte Symmetrie aufweist, da bereits leichte Abweichungen von der Symmetrie schon deutlich wahrnehmbar sind und dauerhafte funktionale sowie auch psychische Belastungen für den Patienten darstellen können.

Bei der Planung der Herrichtung bzw. Rekonstruktion der Knochen- und Weichteilstruktur des Gesichts des Patienten wird üblicherweise zunächst die Symmetrieebene des Gesichts ermittelt. Hierfür wird die geometrische Situation des Gesichts mittels optischer Verfahren erfasst, z.B. auf der Basis von Musterprojektion oder Laser-Abstandsmessung. Aus der somit ermittelten Oberflächenstruktur des Gesichts kann mit unterschiedlichen Verfahren die Symmetrieebene automatisch bestimmt werden. In einem anschließenden Schritt wird die Oberflächenstruktur beider von der Symmetrieebene getrennten Seiten ermittelt, miteinander verglichen und somit Differenzen ermittelt. Dies erfolgt i.d.R. mittels aufwändiger CAD-Programme, die nur für besonders geschulte Benutzer konzipiert sind.

Des Weiteren sind Systeme für die Navigation auf Basis von Knochenstrukturen bekannt und werden z.B. bei chirurgischen Eingriffen an der Wirbelsäule oder im HNO-Bereich eingesetzt. Derartige Systeme umfassen i.d.R. ein Lageerfassungssystem zur Erfassung der Lage von medizinischen Instrumenten sowie des Patienten in einem Referenzkoordinatensystem und ein Führungssystem zur Bereitstellung von Führungsdaten zum Navigieren der chirurgischen Instrumente. Durch das Koordinieren der Führung der medizinischen Instrumente entlasten herkömmliche Navigationssysteme den Chirurgen erheblich, da diese Aufgabe ein großes Maß an räumlicher Vorstellungskraft erfordert. Demnach wird das Operationsrisiko gesenkt und das Operationsergebnis verbessert.

In bekannten Navigations- bzw. Lageerfassungssystemen basiert die Bestimmung der Instrumenten- bzw. Patientenlage meist auf optischen oder elektromagnetischen Verfahren, wobei Systeme mit optischen Verfahren weiter verbreitet sind. Bei üblichen elektromagnetischen Verfahren wird die Lage der medizinischen Instrumente in einem von einem Feldgenerator erzeugten, alternierenden elektromagnetischen Feld über die Stärke von Strömen gemessen, die in einem Lagesensor, der an dem medizinischen Instrument angeordnet ist, induziert werden. Hierfür geeignete Lagesensoren weisen bekanntermaßen mehrere, insbesondere drei Spulen auf. Allerdings haben elektromagnetische Lageerfassungssysteme den Nachteil, dass mögliche Störquellen, wie z.B. aktive chirurgische Instrumente, die bei Betrieb ebenfalls ein elektromagnetisches Feld emittieren, detektiert und kompensiert werden müssen.

Bekannte Mess- und Navigationssysteme haben allesamt den Nachteil, dass sie relativ hohe Anschaffungskosten verursachen, oftmals eine hohe Komplexität aufweisen und nur von speziell ausgebildeten Fachkräften bedient werden können. Ferner sind diese Systeme oftmals umständlich in der Handhabung und bieten somit eine Vielzahl von potenziellen Fehlerquellen. Darüber hinaus benötigen diese Systeme allesamt Bilddaten des Patienten und erfordern somit eine lange Vorbereitungszeit und können ggf. zu einer zusätzlichen Strahlenbelastung des Patienten führen.

Ein Verfahren zum Registrieren von zu einem ersten Datensatz gehörenden Informationen mit einem zweiten Datensatz ist aus der US 2012/0016269 A1 bekannt. WO 01/35842 offenbart ein Verfahren zum Bestimmen von Reduktionsparametern für eine Knochenrepositionsvorrichtung.

Somit ist es die Aufgabe der vorliegenden Erfindung, ein Verfahren sowie ein System zur Planung und Durchführung der Navigation bei der Herrichtung symmetrischer Körperteile eines Patienten bereitzustellen, das die obigen Nachteile nicht aufweist.

Erfindungsgemäß wird diese Aufgabe durch ein medizinisches System zur Unterstützung der Planung und/oder Durchführung der Rekonstruktion üblicherweise symmetrischer Körperteile gelöst. Das System umfasst mindestens eine Messeinheit zum Vermessen einer ersten Fläche sowie einer zweiten Fläche zum Ermitteln einer Topografie der ersten Fläche und der zweiten Fläche. Hierbei kann es sich um Flächen bzw. Oberflächen eines Patienten handeln, wobei die Flächen ausgewählt werden das Kriterium zu erfüllen, dass die erste Fläche bei einem gesunden Menschen üblicherweise im Wesentlichen spiegelbildlich zur zweiten Fläche ist. Ferner ist eine Speichereinheit zum Speichern der ermittelten Topografien der ersten Fläche und der zweiten Fläche des Patienten vorgesehen. Das System weist darüber hinaus eine Auswerteeinheit zum Erstellen eines Spiegelbilds der gespeicherten Topografie der ersten Fläche und zur Berechnung von Abweichungen der gespeicherten Topografie der zweiten Fläche zu dem Spiegelbild der gespeicherten Topografie der ersten Fläche sowie eine Steuereinheit zur Ausgabe von Führungsinformationen für mindestens ein medizinisches Instrument aufgrund der berechneten Abweichungen der Topografie der zweiten Fläche zu dem Spiegelbild der gespeicherten Topografie der ersten Fläche, um die Topografie der zweiten Fläche gemäß dem Spiegelbild der Topografie der ersten Fläche zu rekonstruieren auf. Mit einem erfindungsgemäßen System ist eine Topografie von zwei Symmetriehälften normalerweise symmetrischer Körperteile ermittelbar. Weist eine zu behandelnde Symmetriehälfte eine IST-Topografie auf, die Asymmetrien zur entsprechenden intakten Symmetriehälfte aufweist, kann zur Herstellung bzw. Rekonstruktion der Symmetrie der zu behandelnden Symmetriehälfte durch Spiegeln der Topografie der entsprechenden intakten Symmetriehälfte eine SOLL-Topografie ermittelt werden. Aus den Abweichungen der IST-Topografie zur SOLL-Topografie sind Führungsinformationen zur Steuerung mindestens eines medizinischen Instruments generierbar.

Vorzugsweise weist das medizinische System eine Datenübertragungsschnittstelle zum Übermitteln der von der Messeinheit ermittelten Topografiedaten einer Oberfläche an die Auswerteeinheit und/oder Speichereinheit auf. Die Messeinheit kann dabei kabelgebunden sein oder eine Funkeinrichtung zum Übertragen der Messwerte umfassen.

In einer ersten Ausführungsform der Erfindung umfasst mindestens eine Messeinheit eine mechanische Abtasteinrichtung zum mechanischen Abtasten einer Fläche. Die Abtasteinrichtung kann z.B. einen Pointer mit einem Instrumentengriff und einer Instrumentenspitze zum Abtasten der Fläche oder einzelner Messpunkte umfassen. Die Abtasteinrichtung ist in ein Lageerfassungssystem einbindbar, so dass Lagedaten einer Abtastspitze der Abtasteinrichtung vom Lageerfassungssystem ermittelbar und auf einer Anzeigeeinheit darstellbar sind. Mit einer derartigen Abtasteinrichtung ist die Position einzelner Messpunkte einer Fläche im Koordinatensystem des Lageerfassungssystems erfassbar und somit die Topografie der Fläche durch mechanisches Abtasten ermittelbar.

Besonders bevorzugt umfasst die mechanische Abtasteinrichtung einen Pointer mit einer zumindest teilkugelförmigen Instrumentenspitze. Derartige Instrumentenspitzen haben den Vorteil, dass sie gut über eine Fläche des Patienten gleitend führbar sind, ohne die Fläche zu verletzen. Alternativ, insbesondere zum Abtasten einzelner Messpunkte, sind Pointer mit einer spitzen bzw. kegelförmigen Instrumentenspitze verwendbar, da ein Punkt mit einer derartigen Instrumentenspitze genauer ansteuerbar ist.

In einer zweiten Ausführungsform der Erfindung umfasst mindestens eine Messeinheit eine optische Abtasteinrichtung zum optischen Abtasten einer Fläche. Hierbei werden Lichtwellen emittiert und Reflexionen und/oder Schattenbildungen mit optischen Mitteln erfasst. Aus den Ergebnissen können Rückschlüsse auf die Ausbildung der Topografie der jeweiligen Fläche gezogen werden.

Vorzugsweise umfasst die optische Abtasteinrichtung einen Laser und/oder eine Videoeinrichtung und/oder eine Fotozelle. Besonders bevorzugt sind die von der optischen Abtasteinrichtung ermittelten Bilddaten auf einer Anzeigeeinheit, wie z.B. einem Monitor, darstellbar.

Besonders bevorzugt ist das System ausgebildet, naturgemäße Symmetrien auf Basis von Aufnahmen, beispielsweise Fotos automatisch zu erkennen. Hierfür kann das System eine Erfassungseinheit, beispielsweise eine optische Leseeinheit, aufweisen, die eine Aufnahme, die sowohl die erste Fläche als auch die zu rekonstruierende zweite Fläche umfasst, erfassen kann. Das System kann aus der erfassten Aufnahme eine SOLL-Symmetrielinie ermitteln und in einem zweiten Schritt vorzugsweise auch die Abweichung der IST-Topografie der zweiten Fläche von dessen SOLL-Topografie - dem Spiegelbild der ersten Fläche. Hierfür werden vorzugsweise stereoskopische Aufnahmen, 3D-Aufnahmen oder auch tomografisch erstellte Modelle verwendet.

Die Auswerteeinheit kann ausgebildet sein bei der Berechnung von Abweichungen der gespeicherten Topografie der zweiten Fläche zu dem Spiegelbild der gespeicherten Topografie der ersten Fläche etwaige Schwellungen von Gewebe im Bereich der zweiten Fläche befindlichen Gewebes berücksichtigen. Somit kann erreicht werden, dass eine zu rekonstruierende zweite Fläche postoperativ nach Abschwellen des im Bereich der zweiten Fläche befindlichen Gewebes eine gewünschte Topografie aufweist. Eine Schwellung eines Gewebes kann beispielsweise durch einen Zuschlagswert auf einen optisch oder mechanisch ermittelten Abtastwert berücksichtigt werden. Der Zuschlagswert kann durch eine Höhendifferenz zwischen geschwollenem und abgeschwollenem Zustand des Gewebes gebildet sein. Ein abgeschwollener Zustand kann beispielsweise durch ein Gewebemodell prädiziert werden, das in der Auswerteeinheit hinterlegt sein kann. Das Gewebemodell kann beispielsweise einen Hautspannungskennwert einschließen, der vorzugsweise optisch und/oder mechanisch ermittelt wird. Weiter bevorzugt ist das System ausgebildet, die Topografie naturgemäß symmetrischer Hohlräume zu vermessen, wobei ein erster Hohlraum die erste Fläche und ein zweiter Hohlraum die zweite Fläche umfasst.

Alternativ oder zusätzlich kann die Auswerteeinheit ausgebildet sein beim Erstellen des Spiegelbilds der gespeicherten Topografie der ersten Fläche als intakt definierte Punkte der Topografie der zweiten Fläche zwingend einzubeziehen. Als intakte Punkte können beispielsweise Anschlussstellen eines, insbesondere im oder nahe dem zu rekonstruierenden Gebiet gelegenen Knochens definiert sein. Im Ergebnis kann das Spiegelbild der gespeicherten Topografie der ersten Fläche also imperfekt, insbesondere im Bereich der Anschlussstellen eines Knochens deformiert sein, d.h. bezüglich der Topografie der ersten Fläche asymmetrisch sein. So kann, beispielhaft, bei einer zu rekonstruierenden linken Oberkieferhälfte (in der die zweite Fläche liegt) auf Basis einer rechten Oberkieferhälfte (in der die erste Fläche liegt) mit einer in der anterioren Anlagerungsfläche gelegenen Symmetrieachse das linke Jochbein als intakte Anschlussstelle definiert sein. Da das menschliche Gesicht typischerweise leicht asymmetrisch ist, würde sich beim perfekten Spiegeln der rechten Oberkieferhälfte um die in der anterioren Anlagerungsfläche gelegene Symmetrieachse ein Versatz zwischen linker Oberkieferhälfte und linkem Jochbein ergeben. Durch die Definition des linken Jochbeins als intakte Anschlussstelle wird nun durch die Auswerteeinheit diese Anschlussstelle beim Erstellen des Spiegelbilds der gespeicherten Topografie der ersten Fläche einbezogen. Das Spiegelbild der gespeicherten Topografie der ersten Fläche ist im Bereich dieser Anschlussstelle nunmehr verzerrt, so dass die linke Oberkieferhälfte versatzfrei zum linken Jochbein rekonstruiert werden kann.

In einer vorteilhaften Ausgestaltung der Erfindung ist mindestens ein medizinisches Instrument als Ballonkatheter ausgebildet. Der Ballonkatheter ist der entsprechend den von der Steuereinheit ausgegebenen Führungsinformationen mit einem Arbeitsfluid befüllbar. Der Vorgang des Befüllens des Ballonkatheters ist vorzugsweise von dem medizinischen System automatisch steuerbar, so dass ein manuelles Eingreifen durch den Chirurgen im Wesentlichen nur in Notfällen erforderlich ist. Besonders bevorzugt weist das medizinische System hierfür einen Anschluss für das Arbeitsfluid oder einen entsprechenden Druckerzeuger, wie z.B. einen Kompressor, auf.

Vorzugsweise weist das System mindestens einen Sensor zur Erfassung des Innendrucks und/oder der Ausdehnung des Ballonkatheters auf. Als Sensor zum Ermitteln des Innendrucks des Ballonkatheters sind übliche Drucksensoren für Fluide geeignet. Das Messen der Ausdehnung des Ballonkatheters kann beispielsweise über Dehnungsmessstreifen (DMS) erfolgen. Zum Ermitteln der mehrachsigen Ausdehnung des Ballonkatheters sind mehrere DMS vorgesehen, die jeweils in Richtung einer der zu bestimmenden Dehnungskomponenten angeordnet sind. Besonders bevorzugt weisen mindestens zwei DMS einen Winkel von 90° zueinander auf.

Es ist weiter bevorzugt, dass die von dem Sensor erfassten Daten über eine Sensordatenschnittstelle an die Steuereinheit weiterleitbar sind.

Noch weiter bevorzugt ist die Steuereinheit ausgebildet, das Befüllen des Ballonkatheters unter Berücksichtigung der vom Sensor ermittelten Daten zu steuern. Somit kann die Steuereinheit das Befüllen des Ballonkatheters beenden, wenn der Ballonkatheter eine gewünschte Ausdehnung aufweist.

Vorzugsweise ist die Steuereinheit ausgebildet, eine Befüllgeschwindigkeit des Ballonkatheters in Abhängigkeit von der jeweiligen Ausdehnung bzw. des Innendrucks des Ballonkatheters zu steuern, wobei die Befüllgeschwindigkeit tendenziell mit zunehmender Ausdehnung bzw. mit zunehmendem Innendruck des Ballonkatheters abnehmen soll. Somit soll eine Belastung und etwaige Beschädigung des herzustellenden bzw. zu rekonstruierenden Gewebes reduziert werden.

In einer vorteilhaften Ausgestaltung der Erfindung weist der Ballonkatheter mehrere Kammern auf, wobei die Kammern unabhängig voneinander befüllbar sind. Hierdurch können mit einem Ballonkatheter kompliziertere Geometrien erreicht werden, da die einzelnen Kammern nach Bedarf unterschiedlich befüllbar sind.

Vorzugsweise ist die Auswerteeinheit eingerichtet, Abweichungen der Topografie der zweiten Fläche von der gespiegelten Topografie der ersten Fläche aufgrund einzelner repräsentativer Messpunkte auf der zweiten Fläche zu ermitteln. Eine Vielzahl von Messpunkten, die zwischen zwei repräsentativen Messpunkten angeordnet sind oder deren Lagen im Rahmen der Herstellung bzw. Rekonstruktion nicht verändert werden müssen, müssen demnach nicht vermessen werden. Somit ist die Anzahl der von der zweiten Fläche abzutastenden Messpunkte erheblich reduzierbar. Hierdurch wird auch die Beeinträchtigung bzw. Verletzungsgefahr des Patienten sowie der Arbeitsaufwand beim Abtasten erheblich reduziert.

Besonders bevorzugt ist die Messeinheit ausgebildet, während der Herstellung bzw. Rekonstruktion der zweiten Fläche die Topografie der zweiten Fläche zumindest an repräsentativen Messpunkten zu ermitteln. Somit kann während des Vorgangs der Herstellung bzw. Rekonstruktion der zweiten Fläche der Fortschritt des Eingriffs ermittelt werden.

Weiter bevorzugt passt die Steuereinheit aufgrund der während der Rekonstruktion der zweiten Fläche ermittelten Topografiedaten der zweiten Fläche die Führungsinformationen für mindestens ein medizinisches Instrument an. Bei unvorhersehbaren Abweichungen der Topografie der zweiten Fläche von entsprechenden Sollwerten kann z.B. das Aufweiten des Ballonkatheters durch die Steuereinheit automatisch angehalten werden, um eine Verletzung des Patienten zu verhindern.

In einer vorteilhaften Ausgestaltung der Erfindung ist das medizinische Instrument ein aktives medizinisches Instrument, wobei die Steuereinheit ausgebildet ist, den Betriebszustand des aktiven medizinischen Instruments in Abhängigkeit von der Lage des aktiven medizinischen Instruments zur von der Steuereinheit ermittelten Topografie der zweiten Fläche zu steuern. Somit kann ein aktives medizinisches Instrument, z.B. ein Schneidinstrument angehalten, d.h. zum Beispiel ausgeschaltet werden, wenn die Instrumentenspitze an einer Stelle der ermittelten Topografie der zweiten Fläche angeordnet ist, an der kein Schnitt durchgeführt werden soll. Dementsprechend kann das aktive medizinische Instrument in den aktiven Betriebszustand versetzt werden oder in diesem verweilen, wenn die Instrumentenspitze an einer Stelle der ermittelten Topografie der zweiten Fläche angeordnet wird bzw. ist, an der ein Schnitt durchgeführt werden soll. Eine Drosselung des aktiven medizinischen Instruments bei Annäherung an eine Stelle, an der kein Schnitt durchgeführt werden soll, sowie eine Erhöhung des Betriebszustands des aktiven medizinischen Instruments bei Entfernung von einer solchen Stelle kann ebenfalls vorgesehen sein.

Im Folgenden soll die Erfindung anhand eines Ausführungsbeispiels mit Bezug auf eine Zeichnung näher erläutert werden. Hierbei zeigt:
- Fig. 1: eine Ausführungsform eines medizinischen Systems zur Unterstützung der Planung und/oder Durchführung der Rekonstruktion üblicherweise symmetrischer Körperteile eines Patienten; und
- Fig. 2:: eine vergrößerte Seitenansicht eines Ballonkatheters aus Fig. 1 mit zwei Druckkammern.

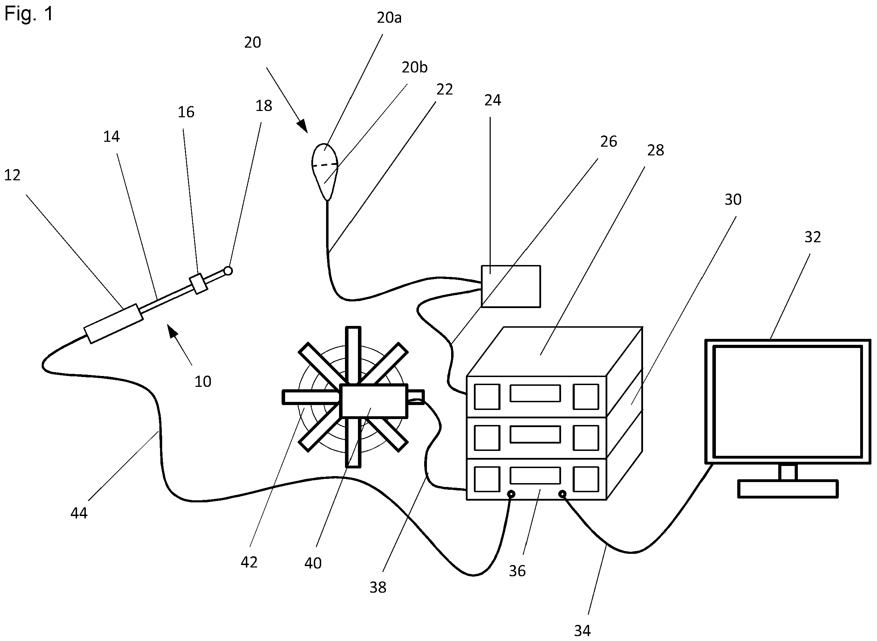

Das in Fig. 1 abgebildete Ausführungsbeispiel eines erfindungsgemäßen medizinischen Systems umfasst eine Auswerteeinheit 36, eine Speichereinheit 30, eine Steuereinheit 28, einen Pointer 10, einen Feldgenerator 40, einen Ballonkatheter 20 mit einem Fluidkompressor 24 sowie eine Anzeigeeinheit 32.

Der Pointer 10 ist über ein Instrumentenkabel 44 mit der Auswerteeinheit 36 verbunden und weist einen proximalen Instrumentengriff 12, eine kugelförmige Instrumentenspitze 18, einen zwischen Instrumentengriff 12 und Instrumentenspitze 18 koaxial zum Instrumentengriff 12 angeordneten Instrumentenschaft 14 sowie einen Instrumentensensor 16, der in dieser Ausführungsform an dem Instrumentenschaft 14 angeordnet ist, auf. Alternativ kann der Instrumentensensor 16 auch an der Instrumentenspitze 18 angeordnet sein. Alternativ zur Kugelform kann die Instrumentenspitze 18 z.B. auch kegel-, tetraeder- oder pyramidenförmig ausgebildet sein, wobei der spitze Teil vorzugsweise in distale Richtung weist. Mit der Instrumentenspitze 18 ist eine Fläche eines im Lageerfassungssystem registrierten Patienten linien- oder punktförmig abtastbar. Zuvor muss der Patient im Lageerfassungssystem registriert werden, damit die Lage des Patienten im Koordinatensystem des Lageerfassungssystems bekannt ist. Hierfür ist ein nicht abgebildeter Patientenlokalisator ortsfest am Patienten angeordnet, wobei in Abhängigkeit der Lage des Patientenlokalisators zum Feldgenerator 40 Ströme im Patientenlokalisator induziert werden. Diese Ströme sind von der Auswerteeinheit 36 messbar. Über einen Abtastvorgang einzelner repräsentativer Punkte am Körper des Patienten mit dem Pointer 10 ist der Patient im Lageerfassungssystem registrierbar.

Während des Abtastvorgangs der Fläche des im Lageerfassungssystem registrierten Patienten wird der Pointer 10 entweder gleitend über die Fläche oder direkt auf einzelne Messpunkte geführt. Die Messpunkte sind vorzugsweise so zu wählen, dass sie besondere Stellen auf der Fläche, wie z.B. hervorstehende Knochen, repräsentieren. Beim gleitenden Führen können Positionsdaten der Instrumentenspitze 18 von der Auswerteeinheit 36 kontinuierlich erfasst und in der Speichereinheit 30 abgespeichert werden. Beim direkten Ansteuern einzelner Messpunkte ist zumindest die Position der Instrumentenspitze 18 beim Erreichen des Messpunktes zu erfassen und abzuspeichern. Über eine entsprechende Software können zwischen zwei Punkten liegende Flächen rechnerisch rekonstruiert werden. Auf diese Weise ist eine Topografie der abgetasteten Fläche des Patienten ermittel- sowie speicherbar.

Die Bestimmung der Position der Instrumentenspitze 18 erfolgt über ein in der Auswerteeinheit 36 integriertes Lageerfassungssystem. Der Feldgenerator 40 ist über ein Generatorkabel 38 mit der Auswerteeinheit 36 verbunden und wird von dieser gesteuert. Der Feldgenerator 40 emittiert ein alternierendes elektromagnetisches Feld 42, das in Abhängigkeit der Lage des Instrumentensensors 16 zum Feldgenerator 40 im Instrumentensensor 16 Ströme induziert. Vorzugsweise weist der Instrumentensensor 16 drei Spulen auf, so dass drei Ströme induziert werden. Die Stromstärken werden von der Auswerteeinheit 36 gemessen und somit die Lage des Instrumentensensors 16 relativ zum Feldgenerator 40 bestimmt. Wenn der Pointer 10 zuvor im Lageerfassungssystem registriert wurde, ist somit auch die Lage der Instrumentenspitze 18 bekannt.

Die Auswerteeinheit 36 kann eine Software aufweisen, mit der symmetrische Flächen erkennbar und Symmetrieachsen ermittelbar sind. Alternativ können Symmetrieachsen auch manuell, z.B. über eine entsprechende Markierung von auf einer Symmetrieachse liegenden Punkten mit Hilfe des Pointers 10 definiert werden. Die Auswerteeinheit 36 ist konfiguriert, die Topografie einer ersten Fläche, die als Referenzfläche bezeichnet wird, über die Symmetrieachse zu spiegeln und das Spiegelbild der Topografie der ersten Fläche mit der Topografie einer zweiten Fläche zu vergleichen, wobei die Topografie der zweiten Fläche im Idealfall bereits der Topografie der gespiegelten ersten Fläche bzgl. Lage und Ausprägung entspricht. Sofern die Topografie der zweiten Fläche von dem Spiegelbild der Topografie der ersten Fläche abweicht kann die Auswerteeinheit 36 die Differenz der beiden Flächen ermitteln und hieraus einen Korrekturwert bestimmen, der an die Steuereinheit 28 weiterleitbar ist. Auf Basis des Korrekturwerts kann die Steuereinheit 28 die Befüllung des Ballonkatheters 20 mittels des Fluidkompressors 24 sowie eines Katheterschlauchs 22, der den Fluidkompressor 24 mit dem Ballonkatheter 20 verbindet, steuern. Der Fluidkompressor 24 ist über ein Kompressorkabel 26 mit der Steuereinheit 28 verbunden.

Auf der Anzeige 32, die über ein Anzeigekabel 34 mit der Auswerteeinheit 36 verbunden ist, sind unterschiedliche Bilddaten vom Patienten, medizinischen Instrumenten sowie Prothesen darstellbar. Hierbei kann es sich sowohl um präoperativ als auch intraoperativ gewonnene Bilddaten handeln, die nebeneinander getrennt auf virtuellen Bildschirmen oder auf einem Bildschirm überlagert abbildbar sind. Insbesondere für die überlagerte Darstellung ist es wichtig, dass alle überlagerten Objekte im Lageerfassungssystem registriert sind, damit Lage und Position der einzelnen Objekte dem Bezugskoordinatensystem der Lageerfassungssystems zugeordnet werden können. Die Anzeige 32 ist in diesem Beispiel ein Monitor.

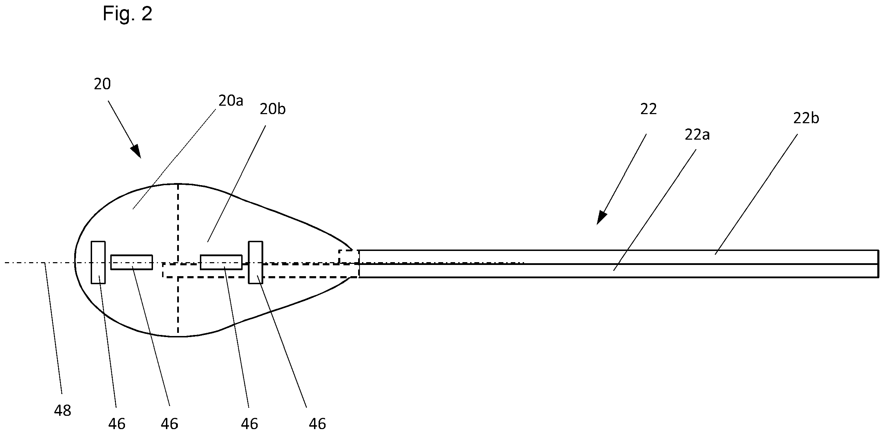

Der Ballonkatheter 20 aus Fig. 1 ist in Fig. 2 in einer vergrößerten Seitenansicht abgebildet und weist eine erste Kammer 20a sowie eine zweite Kammer 20b auf, wobei die erste Kammer 20a über einen ersten Kanal 22a und die zweite Kammer 20b über einen zweiten Kanal 22b des Katheterschlauchs separat voneinander befüllbar sind. Am äußeren Umfang sind an dem Ballonkatheter 20 Dehnungsmessstreifen (DMS) 46 verteilt angeordnet, wobei einige DMS 46 parallel und einige DMS 46 quer zur Längsachse 48 des Ballonkatheters 20 ausgerichtet sind. Über die DMS 46 ist die Aufweitung des Ballonkatheters 20 in Folge der Befüllung bestimmbar. Die DMS sind über ein nicht dargestelltes Kabel mit der Auswerteeinheit 36 und/oder der Steuereinheit 28 verbunden, so dass die Auswerteeinheit 36 bzw. Steuereinheit 28 aufgrund der veränderten Widerstände der DMS 46 die Ausdehnung des Ballonkatheters 20 bestimmen kann. Erreicht die Ausdehnung einen Schwellwert, kann die Steuereinheit 28 das Befüllen des Ballonkatheters 20 bzw. der ersten Kammer 20a oder der zweiten Kammer 20b des Ballonkatheters 20 drosseln oder stoppen.

Der in Fig. 2 gezeigte Ballonkatheter 20 weist eine Eierform mit zwei voneinander getrennten Kammern auf. Alternative Ausführungsformen können eine Vielzahl weiterer Kammern umfassen, die über einen entsprechenden Katheterschlauch 22 vorzugsweise separat voneinander befüllbar sind. Die äußere Form des Ballonkatheters 20 kann je nach Anwendungsfall auch zylindrisch oder im Wesentlichen flächig ausgebildet sein. Neben oder alternativ zu der Verwendung von DMS 46 kann der Ballonkatheter 20 in den Zeichnungen nicht gezeigte Drucksensoren aufweisen, die in der ersten Kammer 20a und/oder der zweiten Kammer 20b angeordnet sind, um den jeweiligen Kammerinnendruck zu erfassen. Analog zu den DMS 46 ist über den von den Drucksensoren ermittelten Kammerinnendruck in Verbindung mit Informationen über die Dehnungseigenschaften des Ballonkatheters 20 die Aufweitung des Ballonkatheters 20 von der Auswerteeinheit 36 automatisch bestimmbar und somit der Befüllvorgang regelbar.

### Bezugszeichen:

- 10: Pointer
- 12: Instrumentengriff
- 14: Instrumentenschaft
- 16: Instrumentensensor
- 18: Instrumentenspitze
- 20: Ballonkatheter
- 20a: erste Kammer
- 20b: zweite Kammer
- 22: Katheterschlauch
- 22a: erster Kanal
- 22b: zweiter Kanal
- 24: Fluidkompressor
- 26: Kompressorkabel
- 28: Steuereinheit
- 30: Speichereinheit
- 32: Anzeigeeinheit
- 34: Anzeigekabel
- 36: Auswerteeinheit
- 38: Generatorkabel
- 40: Feldgenerator
- 42: elektromagnetisches Feld
- 44: Instrumentenkabel
- 46: Dehnungsmessstreifen (DMS)
- 48: Längsachse

## Patentansprüche

1. Medizinisches System zur Unterstützung der Planung und/oder Durchführung der Rekonstruktion üblicherweise symmetrischer Körperteile eines Patienten, umfassend:
- mindestens eine Messeinheit zum Vermessen einer ersten Fläche und einer zweiten Fläche zum Ermitteln einer Topografie der ersten Fläche und der zweiten Fläche;
- eine Speichereinheit (30) zum Speichern der ermittelten Topografien der ersten Fläche und der zweiten Fläche;
- eine Auswerteeinheit (36) zum Erstellen eines Spiegelbilds der gespeicherten Topografie der ersten Fläche und zur Berechnung von Abweichungen der gespeicherten Topografie der zweiten Fläche zu dem Spiegelbild der gespeicherten Topografie der ersten Fläche ;
**gekennzeichnet durch**
- eine Steuereinheit (28) zur Ausgabe von Führungsinformationen für mindestens ein medizinisches Instrument aufgrund der berechneten Abweichungen der Topografie der zweiten Fläche zu dem Spiegelbild der gespeicherten Topografie der ersten Fläche, um die Topografie der zweiten Fläche gemäß dem Spiegelbild der Topografie der ersten Fläche zu rekonstruieren, wobei
- die Messeinheit ausgebildet ist, während der Herstellung bzw. Rekonstruktion der zweiten Fläche die Topografie der zweiten Fläche zumindest an repräsentativen Messpunkten zu ermitteln und
- die Steuereinheit (28) aufgrund der während der Rekonstruktion der zweiten Fläche ermittelten Topografiedaten der zweiten Fläche die Führungsinformationen für das mindestens eine medizinische Instrument anpasst.

2. System nach Anspruch 1,
**gekennzeichnet durch** eine Datenübertragungsschnittstelle zum Übermitteln der von der Messeinheit ermittelten Topografiedaten einer Oberfläche an die Auswerteeinheit (36) und/oder Speichereinheit (30).

3. System nach Anspruch 1 oder 2,
**dadurch gekennzeichnet, dass** das mindestens eine Messeinheit eine mechanische Abtasteinrichtung zum mechanischen Abtasten einer Fläche umfasst, wobei die mechanische Abtasteinrichtung in ein Lageerfassungssystem einbindbar ist, so dass Lagedaten einer Abtastspitze der Abtasteinrichtung vom Lageerfassungssystem ermittelbar und auf einer Anzeigeeinheit (32) darstellbar sind.

4. System nach Anspruch 3,
**dadurch gekennzeichnet, dass** die mechanische Abtasteinrichtung einen Pointer (10) mit einer zumindest teilkugelförmigen Instrumentenspitze (18) umfasst.

5. System nach einem der vorangehenden Ansprüche,
**dadurch gekennzeichnet, dass** mindestens eine Messeinheit eine optische Abtasteinrichtung zum optischen Abtasten einer Fläche umfasst.

6. System nach Anspruch 5,
**dadurch gekennzeichnet, dass** das optische Abtastinstrument einen Laser, eine Videoeinrichtung und/oder eine Fotozelle umfasst und dass die von der optischen Messeinrichtung ermittelten Bilddaten auf einer Anzeigeeinheit (32) darstellbar sind.

7. System nach einem der vorangehenden Ansprüche,
**dadurch gekennzeichnet, dass** mindestens ein medizinisches Instrument als Ballonkatheter (20) ausgebildet ist, der entsprechend den von der Steuereinheit (28) ausgegebenen Führungsinformationen mit einem Arbeitsfluid befüllbar ist.

8. System nach Anspruch 7,
**dadurch gekennzeichnet, dass** das System mindestens einen Sensor zur Erfassung des Innendrucks und/oder der Ausdehnung des Ballonkatheters (20) aufweist.

9. System nach Anspruch 8,
**dadurch gekennzeichnet, dass** die Steuereinheit (28) ausgebildet ist, das Befüllen des Ballonkatheters (20) unter Berücksichtigung der vom Sensor ermittelten Daten zu steuern.

10. System nach Anspruch 9,
**dadurch gekennzeichnet, dass** die Steuereinheit (28) ausgebildet ist, eine Befüllgeschwindigkeit des Ballonkatheters (20) in Abhängigkeit von der jeweiligen Ausdehnung und/oder des Innendrucks des Ballonkatheters (20) zu steuern, wobei die Befüllgeschwindigkeit tendenziell mit zunehmender Ausdehnung bzw. mit zunehmendem Innendruck des Ballonkatheters (20) abnehmen soll.

11. System nach einem der Ansprüche 7 bis 10,
**dadurch gekennzeichnet, dass** der Ballonkatheter (20) mehrere Kammern aufweist, wobei die Kammern unabhängig voneinander befüllbar sind.

12. System nach einem der vorangehenden Ansprüche,
**dadurch gekennzeichnet, dass** die Auswerteeinheit (36) eingerichtet ist, Abweichungen der Topografie der zweiten Fläche von der gespiegelten Topografie der ersten Fläche aufgrund einzelner repräsentativer Messpunkte auf der zweiten Fläche zu ermitteln.

13. System nach einem der vorstehenden Ansprüche,
**dadurch gekennzeichnet, dass** das medizinische Instrument ein aktives medizinisches Instrument ist, wobei die Steuereinheit (28) ausgebildet ist, den Betriebszustand des aktiven medizinischen Instruments in Abhängigkeit von der Lage des aktiven medizinischen Instruments zur von der Steuereinheit (28) ermittelten Topografie der zweiten Fläche zu steuern.

## Claims

1. A medical system for assisting in planning and/or performing the reconstruction of usually symmetrical body parts of a patient, comprising:
- at least one measuring unit for measuring a first area and a second area in order to establish a topography of the first area and the second area;
- a storage unit (30) for storing the established topographies of the first area and the second area;
- an evaluation unit (36) for establishing a mirror image of the stored topography of the first area and for calculating deviations between the stored topography of the second area and the mirror image of the stored topography of the first area
**characterized in that**
- a control unit (28) for outputting guidance information for at least one medical instrument on the basis of the calculated deviations in the topography of the second area from the mirror image of the stored topography of the first area in order to reconstruct the topography of the second area in accordance with the mirror image of the topography of the first area, wherein
- the measuring unit is embodied to establish the topography of the second area, at least at representative measurement points, during the production or reconstruction of the second area, and
- that the control unit (28) adapts the guidance information for at least one medical instrument on the basis of the topography data of the second area established during the reconstruction of the second area.

2. The system as claimed in claim 1,
**characterized by** a data transmission interface for transmitting the topography data of a surface established by the measuring unit to the evaluation unit (36) and/or storage unit (30).

3. The system as claimed in claim 1 or 2,
**characterized in that** the at least one measuring unit comprises a mechanical sensing device for mechanically sensing an area, wherein the mechanical sensing device can be integrated in a position detection system such that position data from a sensing tip of the sensing device are establishable by the position detection system and displayable on a display unit (32).

4. The system as claimed in claim 3,
**characterized in that** the mechanical sensing device comprises a pointer (10) with an at least partly spherically shaped instrument tip (18).

5. The system as claimed in one of the preceding claims,
**characterized in that** at least one measuring unit comprises an optical sensing device for optically sensing an area.

6. The system as claimed in claim 5,
**characterized in that** the optical sensing device comprises a laser, a video device and/or a photocell and **in that** the image data established by the optical sensing device are displayable on a display unit (32).

7. The system as claimed in one of the preceding claims,
**characterized in that** at least one medical instrument is embodied as a balloon catheter (20) which, in accordance with the guidance information output by the control unit (28), is fillable with a work fluid.

8. The system as claimed in claim 7,
**characterized in that** the system has at least one sensor for detecting the internal pressure and/or the extent of the balloon catheter (20).

9. The system as claimed in claim 8,
**characterized in that** the control unit (28) is embodied to control the filling of the balloon catheter (20) taking into account the data established by the sensor.

10. The system as claimed in claim 9,
**characterized in that** the control unit (28) is embodied to control a filling speed of the balloon catheter (20) in a manner dependent on the respective extent and/or on the internal pressure of the balloon catheter (20), wherein the filling speed is tendentiously intended to decrease with increasing extent or with increasing internal pressure of the balloon catheter (20).

11. The system as claimed in one of claims 7 to 10,
**characterized in that** the balloon catheter (20) has a plurality of chambers, wherein the chambers are fillable independently of one another.

12. The system as claimed in one of the preceding claims,
**characterized in that** the evaluation unit (36) is configured to establish deviations in the topography of the second area from the mirrored topography of the first area on the basis of individual representative measurement points on the second area.

13. The system as claimed in one of the preceding claims,
**characterized in that** the medical instrument is an active medical instrument, wherein the control unit (28) is embodied to control the operating state of the active medical instrument in a manner dependent on the position of the active medical instrument from the topography of the second area established by the control unit (28).

## Revendications

1. Système médical pour concevoir ou effectuer plus facilement la reconstruction de parties du corps habituellement symétriques d'un patient, comprenant :
- au moins une unité de mesure pour mesurer une première surface et une deuxième surface afin de déterminer une topographie de la première surface et de la deuxième surface;
- une unité (30) de mémoire pour mémoriser les topographies déterminées de la première surface et de la deuxième surface;
- une unité (36) d'exploitation pour établir une image, comme en un miroir, de la topographie mémorisée de la première surface et pour calculer des écarts de la topographie mémorisée de la deuxième surface à l'image, comme en un miroir, de la topographie mémorisée de la première surface;
**caractérisé par**
- une unité (28) de commande pour émettre des informations de guidage d'au moins un instrument médical, sur la base des écarts calculés de la topographie de la deuxième surface à l'image, comme en un miroir, de la topographie mémorisée de la première surface, afin de reconstruire la topographie de la deuxième surface suivant l'image, comme en un miroir, de la topographie de la première surface, dans lequel
- l'unité de mesure est constituée pour déterminer, pendant la production ou la reconstruction de la deuxième surface, la topographie de la deuxième surface, au moins en des points de mesure représentatifs et
- l'unité (28) de commande adapte, sur la base des données de topographie de la deuxième surface déterminées pendant la reconstruction de la deuxième surface, les informations de guidage du au moins un instrument médical.

2. Système suivant la revendication 1,
**caractérisé par** une interface de transmission de données pour transmettre des données de topographie, déterminées par l'unité de mesure, d'une surface à l'unité (36) d'exploitation et/ou à l'unité (30) de mémoire.

3. Système suivant la revendication 1 ou 2,
**caractérisé en ce que** la au moins une unité de mesure comprend un dispositif de balayage mécanique pour le balayage mécanique d'une surface, le dispositif de balayage mécanique pouvant être incorporé dans un système de détection de position, de manière à ce que des données de position d'une pointe de balayage des dispositifs de balayage puissent être déterminées par le système de détection de position et être représentées sur une unité (32) d'affichage.

4. Système suivant la revendication 3,
**caractérisé en ce que** le dispositif de balayage mécanique comprend un pointeur (10), ayant une pointe (18) d'instrument, au moins en forme de sphère partielle.

5. Système suivant l'une des revendications précédentes,
**caractérisé en ce que** le au moins un dispositif de mesure comprend un dispositif de balayage optique pour le balayage optique d'une surface.

6. Système suivant la revendication 5,
**caractérisé en ce que** l'instrument de balayage optique comprend un laser, un dispositif vidéo et/ou une photopile, et **en ce que** les données d'image, déterminées par le dispositif de mesure optique, peuvent être représentées sur un dispositif (32) d'affichage.

7. Système suivant l'une des revendications précédentes,
**caractérisé en ce qu'**au moins un instrument médical est constitué sous la forme d'un cathéter (20) à ballon, qui peut être rempli d'un fluide de travail conformément aux informations de guidage émises par l'unité (28) de commande.

8. Système suivant la revendication 7,
**caractérisé en ce que** le système a au moins un capteur de détection de la pression à l'intérieur et/ou de l'expansion du cathéter (20) à ballon.

9. Système suivant la revendication 8,
**caractérisé en ce que** l'unité (28) de commande est constituée pour commander le remplissage du cathéter (20) à ballon en tenant compte des données déterminées par le capteur.

10. Système suivant la revendication 9,
**caractérisé en ce que** l'unité (28) de commande est constituée pour commander une vitesse de remplissage du cathéter (20) à ballon en fonction de l'expansion du cathéter (20) à ballon et/ou de la pression à l'intérieur du cathéter (20) à ballon, la vitesse de remplissage devant diminuer tendanciellement au fur et à mesure qu'augmente l'expansion du cathéter (20) à ballon ou que s'élève la pression à l'intérieur du cathéter (20) à ballon.

11. Système suivant l'une des revendications 7 à 10,
**caractérisé en ce que** le cathéter (20) à ballon a plusieurs chambres, les chambres pouvant être remplies indépendamment les unes des autres.

12. Système suivant l'une des revendications précédentes,
**caractérisé en ce que** l'unité (36) d'exploitation est conçue pour déterminer, sur la base de divers points de mesure représentatifs sur la deuxième surface, des écarts de la topographie de la deuxième surface à la topographie, comme en un miroir, de la première surface.

13. Système suivant l'une des revendications précédentes,
**caractérisé en ce que** l'instrument médical est un instrument médical actif, l'unité (28) de commande étant constituée pour commander l'état de fonctionnement de l'instrument médical actif en fonction de la position de l'instrument médical actif par rapport à la topographie, déterminée par l'unité (28) de commande, de la deuxième surface.
